# FASCICULE DE BREVET EUROPEEN

(11) **EP 2 616 015 B1**
(45) Date de publication et mention de la délivrance du brevet: **14.05.2014**
(21) Numéro de dépôt: 11773091.1
(22) Date de dépôt: 15.09.2011
(51) Int. Cl.: A61F 2/40, A61F 2/30

(54) **CUPULE DE PROTHÈSE D'ÉPAULE POURVUE DE NERVURES DE FIXATION**
SCHULTERPROTHESENPFANNE MIT FIXIERRIPPEN
SHOULDER PROSTHESIS CUP INCLUDING SECURING RIBS

(30) Priorité: 17.09.2010 FR 1057435
(43) Date de publication de la demande: 24.07.2013
(73) Titulaire: Duport, Marc, 31400 Toulouse (FR)
(72) Inventeur: Duport, Marc, 31400 Toulouse (FR)
(74) Mandataire: Jeannet, Olivier
(86) Numéro de dépôt international: PCT/FR2011/052117
(87) Numéro de publication internationale: WO 2012/035265

(56) Documents cités:
- EP-A2- 0 538 895
- WO-A2-2008/146124
- DE-B3-102007 032 583
- DE-U1-202006 017 005
- FR-A1- 2 686 503
- FR-A1- 2 928 827
- US-A1- 2004 153 161

## Description

La présente invention se rapporte à une cupule de prothèse, notamment une cupule de prothèse d'épaule.

En France, environ 15 000 prothèses d'épaule sont posées par an, incluant les prothèses totales d'épaules, dites standards, les prothèses inversées, et plus récemment les prothèses de resurfaçage.

Ce chiffre, en nette augmentation ces dernières années, est en particulier dû à l'augmentation du nombre de pathologies pour lesquelles la pose d'une prothèse d'épaule est maintenant indiquée.

Dans ce contexte, la chirurgie prothétique de l'épaule reste une chirurgie très spécialisée, de haute technicité, nécessitant un apprentissage long et l'utilisation d'ancillaires souvent complexes. Une simplification des prothèses et des ancillaires est donc souhaitable afin de faciliter le geste opératoire, de le rendre plus reproductible et donc de diminuer les complications postopératoires. Une telle simplification de l'ancillaire et du geste opératoire aurait également l'avantage de diminuer les coûts de ces opérations de pose de prothèses d'épaule.

Il existe plusieurs types de prothèses d'épaule. Certaines prothèses classiques telles que celle décrite dans US 2004/153161 sont fixées sur l'humérus, grâce à une tige humérale, après une résection conséquente de l'os. L'implantation d'une tige humérale nécessite un évidement important dans l'os pour l'accueillir, diminuant ainsi le capital osseux et fragilisant l'os. La pose d'une telle prothèse est longue et complexe. Après installation de la tige dans l'évidement, l'espace entre l'os et la tige humérale est généralement comblé avec du ciment. Avec le temps, le ciment peut se disloquer et ne plus assurer alors une fixation correcte de la tige.

Il existe également des prothèses de resurfaçage, telles que celle décrite dans FR 2 928 827, qui sont posées sur l'épiphyse de l'humérus après un éventuel ponçage léger de la surface de l'épiphyse. De telles prothèses ne sont pas nécessairement ancrées dans l'os comme décrit précédemment.

Lorsqu'un simple resurfaçage de l'épiphyse n'est pas indiqué, par exemple en cas de fragilité osseuse trop importante, le chirurgien peut choisir de mettre en oeuvre une technique intermédiaire dite de « demi-resurfaçage » ou une partie de l'épiphyse est réséquée. Le chirurgien est alors amené à utiliser une prothèse telle que celle décrite dans le document EP 0538 895, comportant une cupule destinée à remplacer la partie de l'épiphyse réséquée et une vis d'ancrage assure la fixation de la cupule sur l'os.

Il est également connu du document WO 2008/146124 de remplacer la vis d'ancrage décrite précédemment par une quille d'ancrage sensiblement cylindrique. La cupule est alors pourvue d'un plot d'accouplement coopérant par emboîtement avec une cavité, de forme cylindrique complémentaire, ménagée à cet effet dans la quille d'ancrage. Une telle cupule est alors maintenue en place grâce à la pression exercée par la glène. Cependant, la nature de l'accouplement de la cupule et de la quille d'ancrage ne permet pas d'éviter la rotation de la cupule après son implantation, ce qui peut occasionner des problèmes. De plus, la cupule étant simplement emboitée dans la cavité de la quille d'ancrage, l'ancrage de la cupule sur la tête humérale peut s'avérer insuffisant, et ainsi occasionner des douleurs ou un inconfort pour le patient.

Toutes les cupules connues sont spécifiquement adaptées à une technique chirurgicale précise. Le chirurgien doit donc apprendre les différentes techniques et doit disposer de plusieurs ancillaires adaptés à chaque technique. Par ailleurs, si au cours d'une opération le chirurgien rencontre des difficultés, il n'a pas la possibilité d'adapter la prothèse, par exemple en changeant le type de cupule implantée.

La présente invention a pour objet de remédier en tout ou partie aux différents inconvénients cités précédemment.

Les documents FR 2 686 503 A1 et DE 20 2006 017005 U1 décrivent d'autres prothèses existantes. Le préambule de la revendication 1 est basé sur ce que décrit le premier de ces documents.

Dans ce contexte, la présente invention a pour objet de proposer une cupule modulable permettant la mise en oeuvre des différentes techniques (classique, resurfaçage, et semi-resurfaçage), qui préserve le capital osseux du patient et qui garantisse un ancrage correct de la cupule, tout en prévenant une rotation de celle-ci après l'opération.

A cet effet, la présente invention se rapporte à une cupule de prothèse, notamment de prothèse d'épaule, comportant une coque ayant sensiblement une forme de calotte sphérique creuse délimitant une surface intérieure concave, la coque présentant des moyens d'ancrage faisant saillie de la surface intérieure, conformés pour pénétrer dans un os et ancrer la cupule dans l'os, caractérisée en ce que les moyens d'ancrage sont conformés pour induire une rotation de la cupule lors de la pénétration des moyens d'ancrage dans l'os.

Ainsi, une cupule selon l'invention permet de garantir un ancrage simple et rapide lorsque la technique de resurfaçage ou de semi-resurfaçage est mise en oeuvre. Les moyens d'ancrage, en pénétrant dans l'os, sont conformés pour imprimer une rotation de la cupule : cette rotation a pour effet de garantir un ancrage uniforme de la cupule, une meilleure résistance à l'arrachement de la cupule et éviter la rotation de la cupule une fois implantée. Les moyens d'ancrage apportent une meilleure résistance à l'arrachement de la cupule selon l'invention.

Selon une forme d'exécution, les moyens d'ancrage comportent au moins une nervure. Chaque nervure augmente l'ancrage de la cupule dans l'os et/ou améliore la résistance à l'arrachement de la cupule. Chaque nervure permet également d'éviter la rotation de la cupule une fois celle-ci mise en place sur un patient. Avantageusement, chaque nervure vient de matière avec la coque et est donc facile à réaliser.

Selon une possibilité, chaque nervure présente au moins une arrête présentant une portion sensiblement hélicoïdale. Une telle portion sensiblement hélicoïdale est simple à réaliser et peut se comporter dans l'os de la même manière qu'un filetage.

Selon une caractéristique, les moyens d'ancrage sont conformés pour induire une rotation de la cupule d'environ 10° lors de l'impaction.

Selon une possibilité, chaque nervure présente une extrémité située à proximité du bord de la coque.

Selon une forme d'exécution, la cupule comporte en outre un plot d'accouplement s'étendant depuis la surface intérieure, conçu pour permettre l'accouplement de la cupule avec un élément de fixation, notamment une vis d'ancrage ou une quille d'ancrage.

Selon une possibilité, la cupule comporte en outre une paroi, s'étendant depuis la surface intérieure et présentant sensiblement une forme de couronne disposée de façon concentrique autour du plot d'accouplement. Ainsi, une cupule selon l'invention est modulable : Le plot d'accouplement permet, suivant les besoins du chirurgien, d'accoupler la cupule à un élément de fixation. La paroi permet, d'une part, d'ancrer directement dans l'os la cupule pour améliorer sa fixation et, d'autre part, suivant les besoins, d'accoupler la cupule avec un organe intermédiaire présentant une forme de portion de couronne et adapté pour se loger dans le logement délimité entre ladite paroi et le plot d'accouplement. Un tel organe intermédiaire peut être, par exemple, un organe d'accouplement pour accoupler la cupule avec une vis d'ancrage ou encore avec une bague intermédiaire permettant d'espacer la cupule de l'os pour une meilleure adaptation de la prothèse sur le patient. La paroi permet donc au chirurgien de choisir la technique qu'il va utiliser pour fixer la cupule sur le patient et éventuellement de changer de technique en cours d'opération. Compte tenu de la compatibilité possible entre la quille d'ancrage et l'organe intermédiaire, le chirurgien a également la possibilité d'utiliser les deux en même temps.

Selon une caractéristique de l'invention, la paroi est crénelée. Une paroi crénelée permet d'améliorer l'ancrage de la cupule dans l'os. Le crénelage permet également d'éviter la rotation de la cupule après son insertion dans l'os. Enfin, la paroi crénelée empêche la rotation d'un organe intermédiaire éventuellement utilisé.

Selon une forme d'exécution, la paroi présente une épaisseur diminuant en éloignement de la surface intérieure. La diminution de l'épaisseur de la paroi en éloignement de la surface intérieure confère à la paroi une capacité de pénétration dans l'os améliorée.

Selon une forme d'exécution, la paroi délimite avec le plot d'accouplement un logement configuré pour accueillir un organe intermédiaire présentant une surface extérieure sensiblement tronconique.

Selon une possibilité, la coque présente en outre au moins une encoche disposée sur le bord de la coque. Une telle encoche est utile pour la fixation d'un outil destiné à maintenir la cupule en position et à induire une rotation de 10° lors de l'impaction.

L'invention sera bien comprise à l'aide de la description détaillée qui est exposée ci-dessous en regard du dessin annexé, représentant à titre d'exemple non limitatif une forme de réalisation d'une cupule, dans lequel :
La figure 1 est une vue schématique en perspective d'une cupule ;
la figure 2 est une vue schématique en perspective de la cupule de la figure 1 accouplée avec une quille d'ancrage ;
la figure 3 est une vue schématique en perspective de la cupule de la figure 1 accouplée avec une bague intermédiaire et avec une quille d'ancrage ;

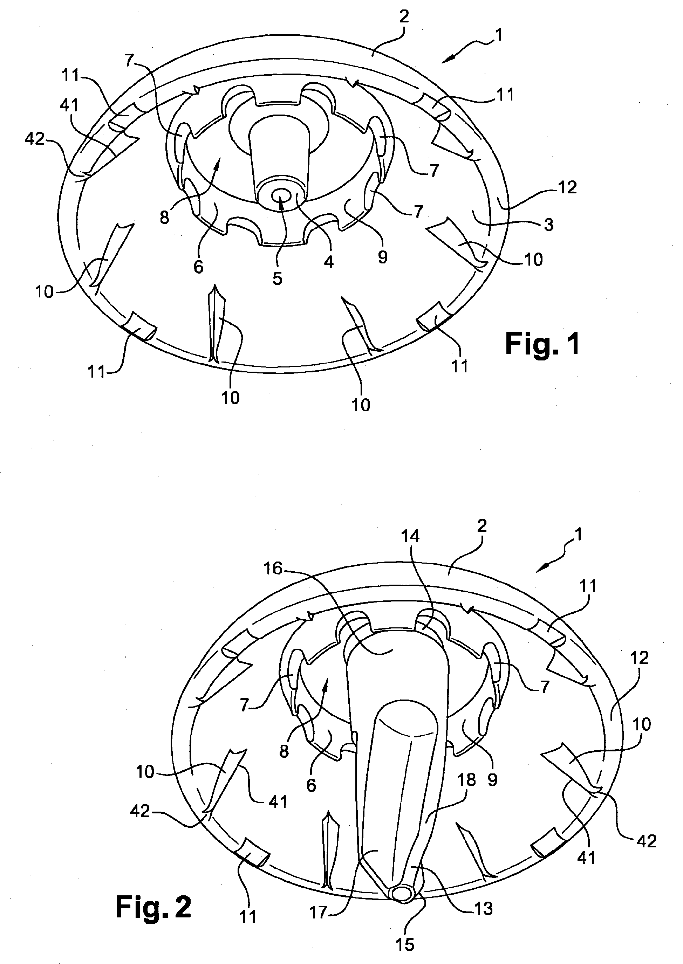

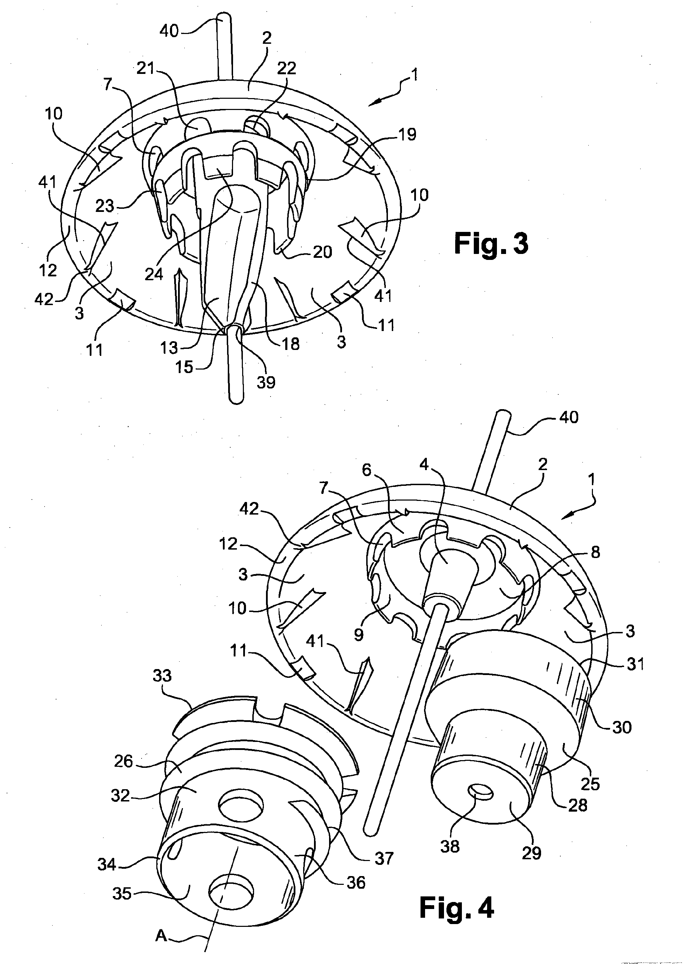

la figure 4 est une vue schématique en perspective de la cupule de la figure 1, d'un organe d'accouplement et d'une vis d'ancrage pour la fixation de la prothèse. Une cupule 1 de prothèse, illustrée à la figure 1, comporte une coque 2 ayant sensiblement une forme de calotte sphérique creuse. La coque 2 délimite une surface intérieure 3 concave et présente un plot d'accouplement 4 qui s'étend depuis cette surface intérieure 3 au niveau du pôle de la coque 2. Le plot d'accouplement 4 est, par exemple, muni d'un orifice traversant 5 s'étendant suivant l'axe de la calotte sphérique. La cupule 1 comporte en outre une paroi 6 qui s'étend depuis la surface intérieure 3 et qui présente une forme sensiblement tubulaire. Cette paroi 6 comporte huit créneaux 7 ménagés à l'extrémité libre de celle-ci. Il est bien évident que le nombre de créneaux 7 peut être adapté en fonction des besoins ou de la taille de la cupule 1. De même la forme de chaque créneau 7 est adaptable et peut être, par exemple, arrondie, comme illustrée à la figure 1, ou rectangulaire. Bien entendu, la profondeur de chaque créneau 7 peut varier jusqu'à être sensiblement égale à la hauteur de la paroi 6. L'épaisseur de la paroi 6 diminue en éloignement de la surface intérieure 3. La paroi 6 est disposée de façon concentrique avec le plot d'accouplement 4 de sorte qu'ils délimitent un logement 8 ayant sensiblement une forme de couronne. La face 9 de la paroi 6 disposée en regard du plot d'accouplement 4 présente une forme tronconique femelle.

La cupule 1 illustrée à la figure 1 comporte également des moyens d'ancrage faisant saillie de la surface intérieure 3 conformés pour pénétrer dans l'os en induisant une rotation de la cupule 1 lors de la pénétration desdits moyens d'ancrage. De tels moyens d'ancrage comportent, par exemple, huit nervures 10 ménagées sur la surface intérieure 3 et venant de matière avec la cupule 1. Il est bien évident que le nombre de nervures 10 peut être adapté en fonction des besoins ou de la taille de la cupule 1. Chaque nervure 10 s'étend selon un axe longitudinal propre, désaxé par rapport à l'axe de la calotte sphérique. Chaque nervure 10 présente une arrête 41, disposée à l'extrémité libre de la nervure 10, présentant une portion 42 sensiblement hélicoïdale. La portion 42 sensiblement hélicoïdale de l'arrête 41 est choisie pour que la pénétration dans l'os de celle-ci, lors de l'ancrage de la cupule 1, facilite une rotation de 10° de la cupule 1 lors de l'impaction. Enfin, chaque nervure 10 est disposée de manière à présenter une extrémité située à proximité du bord 12 de la coque 2. Avantageusement, chacune des extrémités située à proximité du bord 12 de la cupule 1 est tronquée de sorte à former harpon afin d'augmenter la résistance à l'arrachement de la cupule 1.

Enfin, la cupule 1, illustrée à la figure 1, comporte, par exemple, quatre encoches 11 disposées sur le bord 12 de la coque 2.

Un chirurgien peut implanter la cupule 1 de plusieurs manières détaillées ci-après, suivant ses besoins.

Le chirurgien a tout d'abord la possibilité d'utiliser une quille 13 d'ancrage, illustrée à la figure 2, pour implanter la cupule 1, par exemple dans le cadre d'un resurfaçage de l'épiphyse de l'os.

La quille 13 d'ancrage s'étend suivant un axe longitudinal entre une première extrémité 14, destinée à coopérer avec la cupule 1, et une deuxième extrémité 15, destinée à coopérer avec l'os. Elle présente une forme générale tronconique. La quille 13 d'ancrage comporte une première portion 16 conçue pour permettre l'accouplement de la cupule 1. Cette première portion 16 présente un orifice d'accouplement, non représenté, s'étendant selon l'axe longitudinal de la quille 13 et débouchant sur la première extrémité 14. La forme de cet orifice d'accouplement est complémentaire de la forme du plot d'accouplement 4 de la cupule 1, de manière à permettre un emboitement de celui-ci dans l'orifice d'accouplement de la quille 13, pour accoupler la cupule 1 et la quille 13. De façon avantageuse, le plot d'accouplement 4 et l'orifice d'accouplement sont tronconiques. Par exemple, le plot d'accouplement 4 forme un cône morse mâle et l'orifice d'accouplement de la quille 13 est un cône morse femelle complémentaire.

La quille 13 comporte également une deuxième portion 17 conçue pour ancrer la quille 13 dans un os. Cette deuxième portion 17 comporte à cet effet trois arrêtes hélicoïdales 18 permettant à la quille 13 de pénétrer dans l'os à la manière d'un foret en induisant une rotation de la quille 13. Cette rotation de la quille 13 favorise son ancrage et augmente la résistance à l'arrachement de ladite quille 13. Avantageusement, la quille 13 est conformée pour induire une rotation de la cupule 1 dans le même sens de rotation que chaque nervure 10.

Lorsque la quille 13 d'ancrage est utilisée pour fixer la cupule 1, le chirurgien fixe dans un premier temps la quille 13 dans l'épiphyse de l'os puis accouple la cupule 1 sur celle-ci en insérant le plot d'accouplement 4 dans l'orifice d'accouplement de la quille 13. La paroi 6 et les nervures 10 sont alors enfoncées dans l'épiphyse de l'os pour assurer un ancrage supplémentaire de la cupule 1.

La paroi 6 présente une hauteur n'excédant pas 10% du rayon de la calotte sphérique. Par exemple, la hauteur de la paroi 6 est inférieure à 7 mm pour un rayon de la calotte sphérique d'environ 100 mm. Lorsque l'épiphyse de l'os a été partiellement ou totalement réséquée, la hauteur de la paroi 6 peut s'avérer insuffisante pour être ancrées dans l'os. Le chirurgien a alors la possibilité d'utiliser une bague intermédiaire 19 illustrée à la figure 3. Une telle bague intermédiaire 19 présente un corps tubulaire 20 s'étendant selon un axe longitudinal entre deux extrémités opposées. Le corps tubulaire 20 comprend une portion d'accouplement 21 ayant une forme adaptée pour être insérée dans le logement 8 de la cupule 1, de sorte que la surface extérieure 22 de cette portion d'accouplement 21 soit en contact avec la face 9 de la paroi 6 disposée en regard du plot d'accouplement 4, pour assurer un accouplement sans jeu de la cupule 1 et de la bague intermédiaire 19. La portion d'accouplement 21 peut éventuellement comporter une butée, non représentée, faisant saillie de la surface extérieure 22, ladite butée étant destinée à coopérer avec un des créneaux 7 de la cupule 1 pour bloquer la rotation de la bague intermédiaire 19 dans le logement 8.

La bague intermédiaire 19 présente également une portion d'ancrage 23 comportant une paroi d'extension 24 ayant une forme semblable à la paroi 6. Ainsi la paroi d'extension 24 constitue un prolongement de la paroi 6 et est destinée à être ancrée dans l'os de la même manière que la paroi 6. Ainsi, la bague intermédiaire 19 peut être ancrée dans l'os, la cupule 1 étant accouplée sur celle-ci. Lorsque la bague intermédiaire 19 est accouplée avec la cupule 1, l'extrémité libre de la bague intermédiaire 19 se situe à une distance du pôle de la calotte sphérique de l'ordre de 100% du rayon de celle-ci. Ainsi, l'extrémité libre de la bague intermédiaire 19 affleure le bord 12 de la coque 2 de sorte que la bague intermédiaire 19 présente une hauteur maximale pour favoriser l'ancrage de la cupule 1 tout en permettant d'ôter complètement la cupule 1 en sciant l'os au moyen d'une scie guidée par le bord 12 de la coque 2.

Le chirurgien peut également utiliser un organe d'accouplement 25 et une vis 26 d'ancrage pour la fixation de la cupule, illustrés à la figure 4.

L'organe d'accouplement 25 comporte une première portion 27 de forme sensiblement tronconique présentant une portion de montage intermédiaire mâle 28 formant un cône morse mâle 29. L'organe d'accouplement 25 comporte également une deuxième portion tubulaire 30 munie d'une cavité, non représentée, sensiblement tronconique délimitant une portion de montage intermédiaire femelle. La portion de montage intermédiaire femelle forme un cône morse femelle, non représenté, dimensionné pour coopérer par complémentarité de forme avec le plot d'accouplement 4 de la cupule 1. Selon la forme d'exécution représentée sur les figures, l'organe d'accouplement 25 présente un cône morse mâle 29 et un cône morse femelle dont les axes de symétrie respectifs sont confondus. De façon alternative et non représentée, le cône morse mâle 29 et le cône morse femelle peuvent présenter des axes de symétries respectifs distincts décalés et/ou non parallèles. La surface extérieure 31 de la deuxième portion tubulaire 30 a une forme adaptée pour être insérée dans le logement 8 de la cupule 1, de sorte que la surface extérieure 31 de cette deuxième portion tubulaire 30 soit en contact avec la face 9 de la paroi 6 disposée en regard du plot d'accouplement 4, pour assurer un accouplement sans jeu de la cupule 1 et de l'organe d'accouplement 25.

La vis 26 d'ancrage, illustrée à la figure 4, comporte un corps tubulaire 32 s'étendant selon un axe longitudinal A entre une extrémité proximale 33, destinée à coopérer, directement ou indirectement, avec la cupule 1 ou l'organe d'accouplement 25, et une extrémité distale 34 opposée, conçue pour être fixée dans l'épiphyse de l'os. Le corps tubulaire 32 délimite une surface périphérique intérieure 35, illustrée à la figure 4, et une surface périphérique extérieure 36 pourvue de moyens de vissage, réalisés sous la forme d'un filetage 37, pour permettre le vissage de la vis 26 dans l'os.

La surface périphérique intérieure 35 délimite une portion de montage femelle, non représentée, qui s'étend sensiblement à partir de l'extrémité proximale 33 et qui est dimensionnée pour coopérer par complémentarité de forme avec le cône morse mâle 29 de l'organe d'accouplement 25. Dans une forme d'exécution non représentée sur les figures, la portion de montage femelle est dimensionnée pour coopérer par complémentarité de forme avec le plot d'accouplement 4 de la cupule 1 et pour être directement insérée dans le logement 8.

Ainsi, la vis 26 d'ancrage décrit ci-dessus permet de fixer la cupule 1. Lorsque le corps tubulaire 32 de la vis 26 est fixé dans l'os, l'ancrage correct de la vis 26 est assuré grâce au filetage 37 et la procédure de pose est simplifiée, puisque l'accouplement de la cupule 1 sur la vis 26 directement ou indirectement avec l'organe d'accouplement 25 s'effectue par simple emboitement.

Afin de faciliter le positionnement de la cupule 1 et de la quille 13 d'ancrage ou de l'organe d'accouplement 25, ces derniers comportent chacun un orifice traversant 38, 39 s'étendant selon l'axe longitudinal respectivement de l'organe d'accouplement 25 ou de la quille d'ancrage 13. Ainsi, quelque soit la méthode utilisée pour implanter la cupule 1, le chirurgien a la possibilité d'utiliser une tige 40, illustrée aux figures 3 et 4, pour aligner la cupule 1 et la quille d'ancrage 13 ou l'organe d'accouplement 25. La tige 40 est simplement insérée dans l'orifice traversant 5 de la cupule 1 et dans l'orifice traversant 39 de la quille d'ancrage 13 ou dans l'orifice traversant 38 de l'organe d'accouplement 25. La tige 40 peut être ensuite insérée dans un orifice ménagé à cet effet dans l'os pour garantir l'orientation correcte de la cupule 1.

Ainsi, la cupule 1 selon l'invention décrit ci-dessus présente une grande adaptabilité aux différentes techniques chirurgicales couramment utilisées pour la pose d'une prothèse d'épaule. La paroi 6 et les nervures 10 permettent d'ancrer directement dans l'os la cupule 1. La paroi 6 permet également, suivant les besoins du chirurgien, d'accoupler la cupule 1 à un organe d'accouplement 25 ou à une vis 26 d'ancrage présentant une forme de portion de couronne adaptée pour se loger dans le logement 8 délimité entre la paroi 6 et le plot d'accouplement 4. Les nervures 10, en pénétrant dans l'os, sont conformées pour imprimer une rotation de la cupule 1 : cette rotation a pour effet de garantir un ancrage uniforme de la cupule 1, une meilleure résistance à l'arrachement de la cupule 1 et éviter la rotation de la cupule 1 une fois implantée.

Bien entendu, l'exemple de réalisation évoqué ci-dessus ne présente aucun caractère limitatif et d'autres détails et améliorations peuvent être apportés à la cupule 1 selon l'invention, sans pour autant sortir du cadre de l'invention où d'autres formes de cupule peuvent être réalisées.

## Revendications

1. Cupule (1) de prothèse, notamment de prothèse d'épaule, comportant une coque (2) ayant sensiblement une forme de calotte sphérique creuse délimitant une surface intérieure (3) concave, la coque (2) présentant des moyens d'ancrage faisant saillie de la surface intérieure (3), conformés pour pénétrer dans un os et ancrer la cupule (1) dans l'os, **caractérisée en ce que** les moyens d'ancrage sont conformés pour induire une rotation de la cupule (1) lors de la pénétration des moyens d'ancrage dans l'os.

2. Cupule (1) selon la revendication 1, **caractérisée en ce que** les moyens d'ancrage comportent au moins une nervure (10).

3. Cupule (1) selon la revendication 1 ou 2, **caractérisée en ce que** chaque nervure (10) présente au moins une arrête (41) présentant une portion (42) sensiblement hélicoïdale.

4. Cupule (1) selon l'une des revendications 1 à 3, **caractérisée en ce que** les moyens d'ancrage sont conformés pour induire une rotation de la cupule (1) d'environ 10°.

5. Cupule (1) selon l'une des revendications 2 à 4, **caractérisée en ce que** chaque nervure (10) présente une extrémité située à proximité du bord (12) de la coque (2).

6. Cupule (1) selon l'une des revendications 1 à 5, **caractérisée en ce que** la cupule (1) comporte en outre un plot d'accouplement (4) s'étendant depuis la surface intérieure (3), conçu pour permettre l'accouplement de la cupule (1) avec un élément de fixation, notamment une vis (26) d'ancrage ou une quille (13) d'ancrage.

7. Cupule (1) selon la revendication 6, **caractérisée en ce que** la cupule (1) comporte en outre une paroi (6), s'étendant depuis la surface intérieure (3) et présentant sensiblement une forme de couronne disposée de façon concentrique autour du plot d'accouplement (4).

8. Cupule (1) selon la revendication 7, **caractérisée en ce que** la paroi (6) est crénelée.

## Patentansprüche

1. Prothesenpfanne (1), insbesondere für eine Schulterprothese, die eine Schale (2) umfasst, die im Wesentlichen die Form einer hohlen Kugelkappe aufweist, die eine konkave Innenoberfläche (3) begrenzt, wobei die Schale (2) Verankerungsmittel aufweist, die aus der Innenoberfläche (3) hervorstehen, die geeignet sind, in einen Knochen einzudringen und die Pfanne (1) in dem Knochen zu verankern, **dadurch gekennzeichnet, dass** die Verankerungsmittel geeignet sind, beim Eindringen der Verankerungsmittel in den Knochen eine Rotation der Pfanne (1) zu bewirken.

2. Pfanne (1) nach Anspruch 1, **dadurch gekennzeichnet, dass** die Verankerungsmittel mindestens eine Rippe (10) umfassen.

3. Pfanne (1) nach einem der Ansprüche 1 bis 2, **dadurch gekennzeichnet, dass** jede Rippe (10) mindestens einen Grat (41) aufweist, der einen im Wesentlichen schraubenförmigen Abschnitt (42) aufweist.

4. Pfanne (1) nach einem der Ansprüche 1 bis 3, **dadurch gekennzeichnet, dass** die Verankerungsmittel geeignet sind, eine Rotation der Pfanne (1) von ungefähr 10° zu bewirken.

5. Pfanne (1) nach einem der Ansprüche 2 bis 4, **dadurch gekennzeichnet, dass** jede Rippe (10) ein Ende aufweist, das sich in der Nähe des Randes (12) der Schale (2) befindet.

6. Pfanne (1) nach einem der Ansprüche 1 bis 5, **dadurch gekennzeichnet, dass** die Pfanne (1) überdies ein Kopplungsstück (4) umfasst, das sich ausgehend von der Innenoberfläche (3) erstreckt, das ausgestaltet ist, das Koppeln der Pfanne (1) mit einem Fixierungselement, insbesondere mit einer Verankerungsschraube (26) oder mit einem Verankerungskiel (13) oder einem Verankerungskegel (13), zu ermöglichen.

7. Pfanne (1) nach Anspruch 6, **dadurch gekennzeichnet, dass** die Pfanne (1) überdies eine Wand (6) umfasst, die sich ausgehend von der Innenoberfläche (3) erstreckt und die im Wesentlichen die Form einer Krone oder eines Kranzes aufweist, die konzentrisch um das Kopplungsstück (4) angeordnet ist.

8. Pfanne (1) nach Anspruch 7, **dadurch gekennzeichnet, dass** die Wand (6) gezackt ist.

## Claims

1. Prosthesis cup (1), in particular for a shoulder prosthesis (2), comprising a shell (2) substantially in the shape of a hollow spherical cap (3) defining a concave inner surface, the shell (2) including anchoring means that project from the inner surface (3) and are shaped to penetrate a bone and anchor the cup (1) therein, **characterized in that** the anchoring means are shaped to cause a rotation of the cup (1) as they penetrate the bone.

2. Cup (1) according to claim 1, **characterized in that** the anchoring means include at least one rib (10).

3. Cup (1) according to claim 1 or 2, **characterized in that** each rib (10) has at least one sharp edge (41) with a substantially helical portion (42).

4. Cup (1) according to one of claims 1 to 3, **characterized in that** the anchoring means are shaped to cause the cup (1) to rotate by approximately 10° upon impaction.

5. Cup (1) according to one of claims 2 to 4, **characterized in that** each rib (10) has one end situated near the edge (12) of the shell (2).

6. Cup (1) according to one of claims 1 to 5, **characterized in that** the cup (1) also includes a coupling stem (4) extending from the inner surface (3), designed to allow coupling of the cup (1) with a fastening element, in particular an anchoring screw (26) or an anchoring pin (13).

7. Cup (1) according to claim 6, **characterized in that** the cup (1) also includes a wall (6), extending from the inner surface (3) and substantially in the shape of a ring arranged concentrically around the coupling stem (4).

8. Cup (1) according to claim 7, wherein the wall (6) is crenulated.
